# EUROPÄISCHE PATENTANMELDUNG

(11) **EP 1 764 105 A1**
(43) Veröffentlichungstag der Anmeldung: **21.03.2007**
(21) Anmeldenummer: 05020497.3
(22) Anmeldetag: 20.09.2005
(51) Int. Cl.: A61K 36/00

(54) **Verwendung von Gerbstoffen zur Behandlung der Chemotherapie induzierten Diarrhoe**

(71) Anmelder: Rentschler Arzneimittel GmbH, 88471 Laupheim (DE)
(72) Erfinder: Müller, Berno, Dr. med., 88212 Ravensburg (DE)
(74) Vertreter: Schwabe - Sandmair - Marx

(57) **Zusammenfassung**

Die vorliegende Erfindung betrifft die Verwendung eines pflanzlichen Gerbstoffs oder eines Derivats hiervon als Wirkstoff zur Herstellung eines Arzneimittels zur Prävention oder Therapie der Chemotherapie induzierten Diarrhoe (CiD).

## Beschreibung

Die vorliegende Erfindung betrifft die Verwendung eines pflanzlichen Gerbstoffs oder eines Derivats hiervon als Wirkstoff zur Herstellung eines Arzneimittels zur Prävention oder Therapie der Chemotherapie induzierten Diarrhoe (CiD).

### Stand der Technik

Dank der modernen Chemotherapie können oftmals Leben gerettet werden. Gleichzeitig ist sie die Therapieform, vor welcher Krebspatienten im Allgemeinen am meisten Angst haben. Durch die Nebenwirkungen einer Chemotherapie kann die Lebensqualität der Betroffenen schwerwiegend beeinträchtigt werden. Die in der Chemotherapie verabreichten Zytostatika induzieren oftmals neben anderen Beschwerden wie Übelkeit und Erbrechen, Mukositis und Obstipation, schwere Diarrhoe.

Als Diarrhoe (Durchfall) bezeichnet man die häufige und zu schnelle Entleerung von breiigem bis flüssigem Stuhlgang, der je nach Ursache auch mit Schleim, Eiter oder Blut vermengt sein kann. Aus pathophysiologischer Sicht können vier mögliche Mechanismen (osmotisch, sekretorisch, exudativ oder durch Motilitätsstörungen) für eine chronische oder akute Diarrhoe unterschieden werden. Die CiD wird dem sekretorisch-exudativen Typ zugeordnet. Ursächlich ist hierbei ein gestörtes Gleichgewicht zwischen Absorption und Sektretion, vor allem im Dünndarm. Verschiedene Chemotherapeutika führen zu einer Schädigung der intestinalen Schleimhaut mit Verlust der Epitelzellschicht, allgemeiner Entzündungsreaktion mit Influx von Neutrophilen in die Schleimhaut, eingeschränkter Motilität und oberflächlicher Nekrose. Der Entzündungsprozess und die nekrotischen Schleimhautveränderungen verstärken den Verlust von Wasser und Elektrolyten in das Darmlumen. Der Schweregrad der resultierenden Diarrhoe ist jedoch abhängig von der jeweils verwendeten Substanz und ihrer Wirkungsweise.

Unter den häufig verabreichten Zytostatika verursachen vor allem 5-FU, Methotrexat, Irinotecan oder Topotekan und kombinierte Radio-Chemo-Regime Durchfälle. Dies führt oftmals zu Fieber, Kopfschmerzen, Bauchkrämpfen, Übelkeit, Erbrechen, Gewichtsverlust und Gliederschmerzen. Toxische Todesfälle durch Elektrolytimballancen sind insbesondere bei älteren Patienten und Kindern gefürchtet und müssen klinisch bzw. mittels Laborkontrollen rechtzeitig erkannt und nötigenfalls in der Klinik behandelt werden. Ein erhöhter Kreatinin- und ein erniedrigter Kaliumspiegel sind hierbei die Hauptparameter. Der Ausgleich des Elektrolyt- und Flüssigkeitsmangels stehen an vorderster Front der Maßnahmen. Eine solche Substitution kann mit Fertigpräparaten oder, falls die Möglichkeit dazu besteht, mit Infusionen erfolgen. Jedoch führt eine starke Diarrhoe insbesondere bei älteren Patienten und Kindern oftmals dazu, dass eine Chemotherapie nicht erfolgreich durchgeführt werden kann bzw. abgebrochen werden muss.

Nicht nur der Abbruch oder die notwendige Reduktion einer lebensrettenden Chemotherapie ist eine massive Verschlechterung der Therapie-Aussichten. Die Nebenwirkungsrate und damit die "Quality of Life" wird zudem bei allen momentan angewandten Chemotherapie-Regimen als ein Zielparameter der Effektivität der Therapie herangezogen. Die Verlängerung der Überlebenszeiten und ganz entscheidend die Qualität dieser verlängerten Überlebenszeit tritt somit vermehrt in den Focus der Therapie-Ausrichtung und Optimierung. Weiterhin erhöht die möglicherweise erforderliche stationäre Behandlung der Patienten mit CiD die Kosten der Chemotherapie beträchtlich (George Dranitsaris et al "Estimating the cost of illness in colorectal cancer patients who were hospitalized for severe chemoherapy-induced diarrhea", Can. J. Gastroenterol 2005, Vol. 19, Seite 83).

Die CiD Standartbehandlung basiert auf der Verabreichung hoher Dosen an Loperamid. Diese ist jedoch nicht immer erfolgreich. Ein großer Teil der CiD Patienten zeigt sich resistent gegenüber einer Behandlung mit Loperamid. Barbounis et al (Support Care Cancer (2001) 9: 258-260) schlägt die Behandlung von CiD mit Octreotid vor. Die Verwendung von Octreotid wurde in einer Phase 1 Studie an Patienten getestet, deren CiD sich gegenüber einer Behandlung mit Loperamid resistent zeigte. Die Autoren schlossen aus ihren Ergebnissen, dass Octreotid ein effektiver Wirkstoff gegen die Loperamid resistente Diarrhoe ist, die durch eine Irinotecan basierte Chemotherapie verursacht wird. Als Opioid ist Loperamid für eine längere Behandlung jedoch ungeeignet. Die Behandlung mit Octreotid ist aufgrund der vielfältigen Nebenwirkungen ebenfalls belastend für die Patienten. Dabei spielen nicht nur die zentralnervös bekannten Nebenwirkungen eine Rolle sondern auch die gefürchtete Komplikation des Darmverschlusses bei kompletter Lahmlegung des Darmtraktes, was vor allem bei den ohnehin schon geschwächten Patienten unter Chemotherapie deutlich erhöhte Risikopotentiale bei einer gegebenenfalls notwendigen operativen Intervention nach sich zieht.

Rosenoff (Support Care Cancer 2004 12: 561-570) beschreibt, dass subkutan verabreichtes Octreotid effektiv gegen CiD, der sich gegenüber konventionellen Therapien beständig zeigt, eingesetzt werden kann. Um die täglich nötigen Injektionen zu vermeiden, wird eine mikroverkapselte Langzeit-Formulierung (LAR), die den Wirkstoff Octreotid enthält, vorgeschlagen. Die Formulierung wird einmal monatlich intramuskulär (i.m.) verabreicht.

Aufgabe der vorliegenden Erfindung ist es, eine Wirkstoffklasse bereit zu stellen, die eine höhere Wirksamkeit bei der Behandlung von CiD zeigt, als die konventionellen Wirkstoffe aus dem Stand der Technik. Vorteilhafter Weise sollte es sich hierbei um einen selbst nebenwirkungsarmen Wirkstoff handeln, der in optimaler Weise oral verabreicht werden kann.

### Zusammenfassung der Erfindung

Die oben genannte Aufgabe wird gelöst durch die Verwendung eines pflanzlichen Gerbstoffs oder eines Derivates davon zur Herstellung eines Medikaments zur Therapie der Chemotherapie induzierten Diarrhoe (CiD).

### Genaue Beschreibung der Erfindung

Die vorliegende Erfindung betrifft somit die Verwendung eines pflanzlichen Gerbstoffs oder eines Derivates hiervon als Wirkstoff zur Herstellung eines Arzneimittels zur Prävention oder zur Therapie der CiD.

Unter pflanzlichen Gerbstoffen werden Gerbstoffe verstanden, wie sie in verschiedenen Pflanzenteilen vorkommen z.B. in Blättern (z.B. Tee), Samen (z.B. Kaffee), Beeren, Gallen, Hölzern und so weiter. Bevorzugt sind Gallen wie Chinesische Galle. Weiterhin schließt die Erfindung Derivate von pflanzlichen Gerbstoffen ein. Zum Beispiel kann der natürlich vorkommende Gerbstoff durch zusätzliche chemische Einheiten funktionalisiert z.B. verseift oder verestert werden. Bei den Derivaten kann es sich aber auch um Addukte, vorzugsweise Proteinaddukte handeln. Der pflanzliche Gerbstoff oder dessen Derivat kann aus einer Pflanze gewonnen werden oder synthetisch hergestellt sein. Bevorzugt sind natürliche Gerbstoffe.

Zur Behandlung der akuten Diarrhoe ist die Verwendung von Gerbstoffen bzw. Gerbstoffderivaten wie Tanninen bekannt. Eine akute Diarrhoe wird durch schleimhautreizende Nahrungsmittel (alimentäre, irritative Diarrhoe), Laxanzienabusus, Allergene, Krankheitskeime (Dysenterie, Säuglingsenteritis), Darmentzündungen oder Dysfermentie verursacht. Die CiD unterscheidet sich von der akuten Diarrhoe dadurch, dass sie durch die Anwendung von Zytostika d.h. durch eine Beschädigung des Epithels ausgelöst wird.

Sofern der Gerbstoff oder dessen Derivat eine Gerbsäure ist, kann es sich um die freie Säure, ein Salz davon, ein partiell hydrolysiertes oder verseiftes Produkt, einen Ester der Gerbsäure mit einem C₁-C₄-Alkyl oder ein Proteinaddukt derselben handeln.

Bevorzugt ist der pflanzliche Gerbstoff ausgewählt aus der Gruppe, bestehend aus Catechinen, Gallotanninen, Flavanolen und Leukoanthocyanidinen. Weiterhin bevorzugte Gerbstoffe sind Theaflavin, Epicatechin und Epigallocatechin, Catechu, Quebracho, wie sie zum Beispiel in Wattlerrinde, Mangrovenrinde, Hemmlockrinde, Eichenrinde und Tizera enthalten sind. Phenolglucoside z.B. aus Fichtenrinde sowie Piceatannol sind ebenfalls geeignet.

Besonders bevorzugt sind die Tannine bzw. Gallotannine. Gallotannine haben gemeinsam, dass sie Gallussäurereste in ihrer chemischen Struktur aufweisen, die mit einem zentralen Zucker, vorzugsweise Glucose verestert sind. Das Carboxlat der Gallussäure ist in Formel III gezeigt.

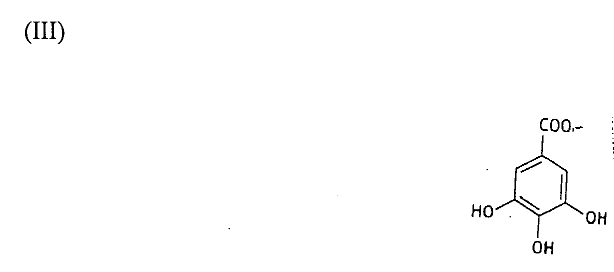

Am stärksten bevorzugt sind die Gallotannine der folgenden Formeln:

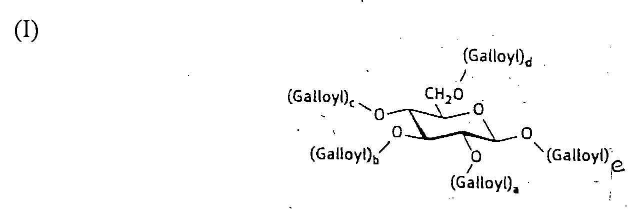

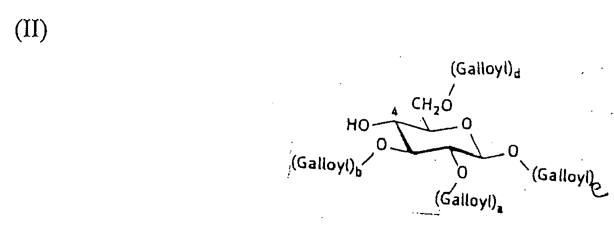

Die zentrale D-Glucoseeinheit ist hierbei über Acetalbrücken mit Gallolylresten verbunden. Unter einem Gallolylrest versteht man einen Rest, der aus einer oder mehreren Gallussäureeinheiten (Gallussäureresten) bestehen kann. Der erste Gallussäurerest bildet über die Carbonsäuregruppe mit einer OH-Gruppe der Glucose eine Acetalbindung. Der Carboxylrest der jeweils folgenden Gallussäure verestert mit einer der Hydroxygruppen der mit dem Zucker verbundenen Gallussäure. Vorzugsweise verestern die weiteren Gallussäurereste mit der Hydroxylgruppe in der Metastellung.

Die Indices a, b, c, d, und e in Formel (I) und (II) sind jeweils unabhängig eine ganze Zahl zwischen 0 und 4, vorzugsweise 0 bis 2, wobei dann, wenn sie 0 sind die Verbindung in dieser Position eine Hydroxylgruppe aufweist. Die Summe von a + b + c+ d + e ist 2 bis 10 vorzugsweise 2 bis 6.

Wenn der Gerbstoff ein Gallotannin der Formel (I) ist, sind a, b, c und d unabhängig voneinander 1 oder 2, e ist 1 und die Summe von a + b + c + d ist 4 bis 6.

Sofern der Gerbstoff ein Gallotannin der Formel (II) ist, sind a, b und c unabhängig voneinander 1 oder 2 und e ist 1. Die Summe von a+ b + d ist dann 2 bis 5. Die Gallotannine der Formeln (I) und (II) werden üblicherweise in Mischung verwendet.

Das Gerbstoff- bzw. Gerbsäurederivat ist vorzugsweise ein Proteinaddukt. Das Protein ist bevorzugt ausgewählt aus der Gruppe, bestehend aus Albuminen, vorzugsweise Serumalbuminen, wie Rinderserumalbumin, Kälberserumalbumin, Ovalbuminen, wie Hühnerovalbumin, Casein, Lactalbumin, Sojaprotein, Hydrolysaten davon und Mischungen derselben.

Bevorzugt sind hierbei Addukte zwischen den erfindungsgemäßen Gerbstoffen mit Ovalbumin oder Mischungen davon. Am meisten bevorzugt ist ein Ovalbuminaddukt eines Gallotannins der Formeln (I) und/oder (II) bzw. ein Tanninalbuminat. Ein entsprechendes Handelsprodukt wird von der Anmelderin unter der Marke Tannacomp ® vertrieben.

Der Wirkmechanismus der erfindungsgemäß verwendeten Gerbstoffe setzt dabei am Schleimhautschutz einer belasteten Darmschleimhaut an. Durch die reversible Vernetzung von Kollagenstrukturen wird eine Schutzmembran gebildet, die eben nicht nur die durch welche Ursachen auch immer hervorgerufene Belastung der Epithelzellschichten reduziert und dem darunter gelegenen Gewebe eine Regenerationsmöglichkeit bietet. Darüber hinaus werden gleichzeitig die für den Elektrolytverlust ursächlichen Erweiterungen der Zell-Zell-Kontakte (tight junctions zu leaky junctions) durch die adstringierenden Eigenschaften reduziert. Dies führt zur Reduktion des Elektrolyt- und Volumenverlustes an der Zelloberfläche.

Die Verabreichung des erfindungsgemäßen Arzneimittels kann in beliebiger Weise erfolgen, sofern das Gerbstoffderivat in ausreichenden Mengen in den Magendarmtrakt gelangt. Bevorzugter Weise ist die Arzneiform zur systemischen, oralen Applikation geeignet.

Die Arzneiform kann ausgewählt sein aus der Gruppe, bestehend aus Tropfen, Tinkturen, Trinklösungen, Tabletten, Kapseln, Mikrotabletten, Pellets, Nanotabletten, Nanopellets, Granulat, Pulver, Dragees, in Kapseln gefüllte Mikrotabletten, Pellets, Nanotabletten, Nanopellets, Granulat und Pulver in Sacchets gefüllte Mikrotabletten, Pellets, Nanotabletten, Nanopellets, Granulat und Pulver, vorzugsweise Tabletten.

Vorzugsweise ist die Arzneiform eine feste orale Arzneiform, die mit einer enterischen Beschichtung versehen sein kann.

Neben den Wirkstoffen können die Arzneimittel der Erfindung die üblichen Hilfsstoffe enthalten. Derartige Hilfsstoffe sind dem Fachmann bekannt. Beispielshaft als Hilfsstoffe zu nennen sind ist hochdisperses Siliciumdioxid, Hypromellose, Lactat, Macrogol 400, Macrogol 6000, Magnesiumstearat, mikrokristalline Cellulose, Natriumcarboxymethylstärke, Talkum, sowie Farbstoffe wie E104, E110, E171 und E172.

Weiterhin kann die Arzneimittelform als weiteren Wirkstoff Alkylacridin oder ein pharmazeutisch annehmbares Salz oder ein Säureaddukt davon enthalten. Die Alkylgruppe ist üblicherweise eine C₁₋₆-Alkylgruppe. Vorzugsweise handelt es sich um eine Methyl-, Ethyl-, Propyl-, Butyl- oder Isobutyl-gruppe. Besonders bevorzugt ist Ethacridin, d.h. die Alkylgruppe ist Ethyl. Bevorzugt ist hierbei, dass das erfindungsgemäße Gerbstoffderivat mit dem Alkylacridin in einer Dosisform formuliert ist.

Sofern das Alkylacridin als Salz oder Addukt vorliegt, so kann es sich um ein Salz oder Addukt einer Säure handeln. Diese ist vorzugsweise ausgewählt aus der Gruppe, bestehend aus HCl, HBr, Kohlensäure, Essigsäure, Milchsäure, Zitronensäure, Weinsäure, Apfelsäure, Oxalsäure, Glykolsäure und Phosphorsäure. Alle pharmazeutisch annehmbaren Säuren können jedoch verwendet werden.

Die tägliche Gesamtdosis an Gerbstoff, die durch Arzneimittelform verabreicht wird, liegt zwischen 100 mg bis 5000 mg vorzugsweise 500 mg bis 4000 mg, am stärksten bevorzugt 1000 bis 4000 mg pro Tag.

Bezogen auf das,Körpergewicht liegt eine sinnvolle erfindungsgemäße Dosierung des Gerbstoffs im Bereich von 2 bis 240 mg, vorzugsweise 5 bis 120 mg, am stärksten bevorzugt 10 bis 55 mg pro kg Körpergewicht pro Tag.

Im Folgenden wird beispielhaft die Zusammensetzung einer Filmtablette (500 mg) angegeben, die zur Behandlung von CiD geeignet ist.

| **Inhalt** | **in mg** | **in Gewichts-%** |
|---|---|---|
| | gesamt 674,09 | gesamt 100% |
| **arzneilich wirksame Bestandteile** | | |
| Tanninalbuminat | 500 | 74,17 |
| Ethacridinacetat-Monohydrat | 50 | 7,42 |
| | | |

| **sonstige Bestandteile** | | |
|---|---|---|
| Hochdisperses Siliciumdioxid | 6,00 | 0,89 |
| Poly(O-carboxymethyl)stärke-Natriumsalz (Typ A) | 26,00 | 3,86 |
| Mikrokristalline Cellulose (Ph. Eur.) | 50,00 | 7,42 |
| Magnesiumstearat [pflanzlich] (Ph.Eur.) | 6,00 | 0,89 |
| Talkum (Ph. Eur.) | 19,00 | 2,82 |
| Macrogol 400 | 1,80 | 0,27 |
| Macrogol 6000 | 1,20 | 0,18 |
| Hypromellose (Substitution Type 2910, Viskosität 3 mPa*s) | 7,00 | 1,04 |
| Chinolingelb, Aluminiumsalz (E 104) | 0,80 | 0,12 |
| Eisen{111}-hydroxid-oxid xH₂O(E110) | 0,24 | 0,04 |
| Gelborange S, Aluminiumsalz (E110) | 2,40 | 0,36 |
| Titandioxid (E171) (Ph. Eur.) | 3,65 | 0,54 |

## Patentansprüche

1. Verwendung eines pflanzlichen Gerbstoffs oder eines Derivats hiervon als Wirkstoff zur Herstellung eines Arzneimittels zur Prävention oder Theraphie der Chemotherapie induzierten Diarrhoe (CiD).

2. Verwendung nach Anspruch 1, worin das Gerbstoffderivat eine Säure, ein Salz, ein partiell hydrolysiertes oder verseiftes Produkt des Gerbstoffes, ein Ester mit einem C₁₋₄-Alkyl oder ein Proteinaddukt ist.

3. Verwendung nach Anspruch 1 oder 2, worin der Gerbstoff ausgewählt ist aus der Gruppe, bestehend aus Catechinen und Gallotanninen natürlichen und synthetischen Ursprungs sowie Mischungen davon.

4. Verwendung nach Anspruch 3, worin der Gerbstoff ausgewählt ist unter Gallotanninen der folgenden Formeln: 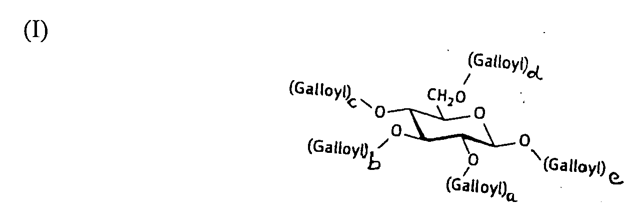 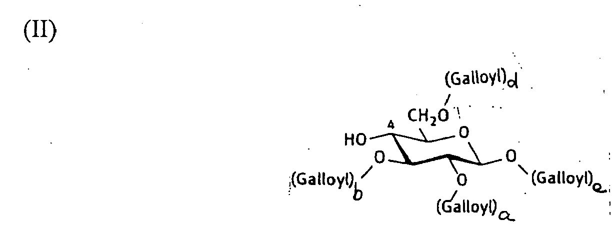 worin
a, b, c, d, und e jeweils unabhängig eine ganze Zahl zwischen 0 und 4, vorzugsweise 0 bis 2 darstellen, wobei dann, wenn sie 0 sind, die Verbindung in dieser Position eine Hydroxylgruppe aufweist,
die Summe von a + b + c + d + e = 2 bis 10, vorzugsweise = 2 bis 6 ist, und (galloyl) für einen Gallussäurerest der Formel 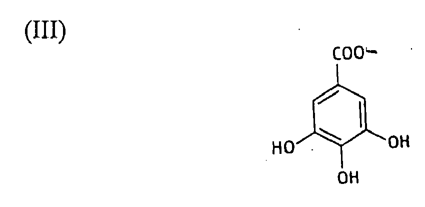 steht, der für a, b, c, d oder e > 1 über einen der Hydroxyreste, vorzugsweise einem Hydroxyrest in meta-Stellung, mit einem weiteren Gallussäurerest verestert ist.

5. Verwendung nach Anspruch 4, worin der Gerbstoff ein Gallotannin der Formel (I) ist, worin a, b, c und d unabhängig voneinander 1 oder 2 sind, e 1 ist und die Summe von a + b + c + d = 4 - 6 ist.

6. Verwendung nach Anspruch 4, worin der Gerbstoff ein Gallotannin der Formel (II) ist, worin a, b und d unabhängig voneinander 1 oder 2 sind, e 1 ist und die Summe von a + b+d=2-5 ist.

7. Verwendung nach Anspruch 3, worin das Gerbstoffderivat ein Proteinaddukt ist, worin das Protein ausgewählt ist aus der Gruppe, bestehend aus Albuminen, vorzugsweise Serumalbuminen wie Rinderserumalbumin, Kälberserumalbumin, Ovalbuminen wie Hühnerovalbumin, Casein, Lactalbumin, Sojaprotein, Hydrolysaten davon und Mischungen derselben.

8. Verwendung nach einem der vorstehenden Ansprüche, worin der Gerbstoff ausgewählt ist aus der Gruppe, bestehend aus Gallotanninen der Formel (I), Gallotanninen der Formel (II), einem Addukt derselben mit Ovalbumin oder Mischungen davon.

9. Verwendung nach einem der vorstehenden Ansprüche, worin die Arzneiform zur systemischen, vorzugsweise oralen Applikation geeignet ist.

10. Verwendung nach Anspruch 9, worin die Arzneiform ausgewählt ist aus der Gruppe, bestehend aus Tropfen, Tinkturen, Trinklösungen, Tabletten, Kapseln, Mikrotabletten, Pellets, Nanotabletten, Nanopellets, Granulat, Pulver, Dragees, Suppositorien, in Kapseln gefüllten Mikrotabletten, Pellets, Nanotabletten, Nanopellets, Granulat und Pulver und in Sacchets gefüllten Mikrotabletten, Pellets, Nanotabletten, Nanopellets, Granulat und Pulver, vorzugsweise Tabletten.

11. Verwendung nach Anspruch 9, worin die Arzneiform eine feste orale Arzneiform und mit einer enterischen Beschichtung versehen ist.

12. Verwendung nach einem der vorstehenden Ansprüche die in Kombination mit einem Alkylacridin oder pharmazeutisch annehmbaren Salz oder Säureaddukt davon erfolgt.

13. Verwendung nach Anspruch 12, worin der Gerbstoff oder dessen Derivat mit dem Alkylacridin in einer Dosisform formuliert ist.

14. Verwendung nach Anspruch 12 oder 13, worin das Alkylacridin ein C₁₋₄-Alkylacridin, vorzugsweise Ethacridin ist.

15. Verwendung nach einem der Ansprüche 12 bis 14, worin das Salz oder Addukt ein Salz oder Addukt einer Säure ist, ausgewählt aus der Gruppe, bestehend aus HCl, HBr, Kohlensäure, Essigsäure, Milchsäure, Zitronensäure, Weinsäure, Äpfelsäure, Oxalsäure, Glykolsäure und Phosphorsäure.

16. Verwendung nach einem der vorstehenden Ansprüche, worin der Gerbstoff in einer Menge von 500 mg bis 4000 mg pro Tag, vorzugsweise 1000 bis 4000 mg pro Tag verabreicht wird.

17. Verwendung nach einem der vorstehenden Ansprüche, worin der Gerbstoff in einer Menge von 5 bis 120 mg pro kg Körpergewicht pro Tag, vorzugsweise 10 bis 55 mg pro kg Körpergewicht pro Tag verabreicht wird.
